# EUROPEAN PATENT APPLICATION

(11) **EP 4 371 544 A1**
(43) Date of publication of application: **22.05.2024**
(21) Application number: 22946043.1
(22) Date of filing: 29.09.2022
(51) Int. Cl.: A61G 7/00

(54) **METHOD AND SYSTEM FOR CONTROLLING DOCKING OF MOBILE SICKBED WITH MEDICAL APPARATUS**

(71) Applicant: Shanghai United Imaging Healthcare Co., Ltd., Shanghai 201807 (CN)
(72) Inventor: LIU, Yongjian, Shanghai 201807 (CN); SONG, Hongyan, Shanghai 201807 (CN); LIU, Lili, Shanghai 201807 (CN)
(74) Representative: Wang, Bo
(86) International application number: PCT/CN2022/122579
(87) International publication number: WO 2024/065395

(57) **Abstract**

A controlling system method docking for a mobile patient bed with a medical device. The method may include: obtaining, based on a docking instruction, positioning information of a target; controlling, based on the positioning information of the target, a movement of the mobile patient bed; and in response to determining that the mobile patient bed moves into a docking region of the medical device, controlling the mobile patient bed to dock with the medical device under a guidance of at least two docking structures. By adopting the embodiments of the present disclosure, an operator may carry a mobile device to move, and control the movement of the patient bed by continuously changing a position of the mobile device, or may enable the patient bed to obtain an automatically planned path through the positioning information of the medical device and to move along the automatically planned path.

## Description

### TECHNICAL FIELD

The present disclosure relates to the field of medical equipment, and in particular relates to a controlling method and system for docking a mobile patient bed with a medical device.

### BACKGROUND

In a practical application of a medical equipment, a patient may often be transferred to different medical equipment positions. For example, when the patient has a magnetic resonance imaging (MRI) examination, it may be necessary to manually push a mobile patient bed forward. When the patient bed is docked with the medical equipment (such as a magnetic resonance equipment), requirements for a docking position may be high, making it difficult for manpower to realize the docking quickly and accurately. Therefore, during a process of the patient being transferred to the MRI for an actual scanning and testing, there may be a waste of time and an inconvenient docking. Therefore, it is desired to provide a controlling method and system for docking a mobile patient bed with a medical device to alleviate a workload of healthcare personnel and improve an efficiency of the docking operation, so as to gain time and resources for the patient's treatment.

### SUMMARY

One of the embodiments of the present disclosure provides a controlling method and system for docking a mobile patient bed with a medical device. The controlling method may include: obtaining, based on a docking instruction, positioning information of a target; controlling, based on the positioning information of the target, a movement of the mobile patient bed; and in response to determining that the mobile patient bed moves into a docking region of the medical device, controlling the mobile patient bed to dock with the medical device under a guidance of at least two docking structures.

In some embodiments, the positioning information of the target may include positioning information of the medical device; and the controlling, based on the positioning information of the target, the movement of the mobile patient bed may include: obtaining an automatically planned path based on the positioning information of the medical device, and moving the mobile patient bed along the automatically planned path; or, moving the mobile patient bed based on the positioning information of the medical device and a preset path.

In some embodiments, the positioning information of the target may include positioning information of a mobile device, and the controlling, based on the positioning information of the target, the movement of the mobile patient bed may include: controlling, based on the positioning information of the mobile device, the mobile patient bed to follow the mobile device.

In some embodiments, the controlling, based on the positioning information of the mobile device, the mobile patient bed to follow the mobile device may include: determining, based on the positioning information of the mobile patient bed and the positioning information of the mobile device, a movement parameter of the mobile patient bed.

In some embodiments, in response to determining that the mobile patient bed is outside a guiding region, the mobile patient bed may be controlled to follow the mobile device based on the positioning information of the mobile device; and in response to determining that the mobile patient bed is moved into the guiding region, the mobile patient bed may be controlled to move based on the positioning information of the medical device.

In some embodiments, in response to determining that the mobile patient bed is outside the guiding region, the mobile patient bed may be controlled to follow the mobile device based on the positioning information of the mobile device; and in response to determining that the mobile patient bed moves into the guiding region, the positioning information of the mobile patient bed may be obtained through a wireless positioning module, and the mobile patient bed may be controlled to move based on the positioning information of the mobile patient bed.

In some embodiments, the obtaining the automatically planned path based on the positioning information of the medical device may include: capturing, by a camera, an optical image including a surrounding of the mobile patient bed; and obtaining, by a path planning module, the automatically planned path based on the optical image.

In some embodiments, the obtaining the automatically planned path based on the positioning information of the medical device may include: sensing, by a plurality of sensors, traffic information around the mobile patient bed; and obtaining, by a path planning module, the automatically planned path based on the traffic information.

In some embodiments, the controlling method may further include: displaying, by a display device, a current movement position of the mobile patient bed.

In some embodiments, the controlling method may further include: in response to determining that the mobile patient bed moves into the docking region of the medical device and a first docking structure on the mobile patient bed starts to dock with a second docking structure on the medical device, controlling the second docking structure on the medical device and the first docking structure on the mobile patient bed to actively drag the mobile patient bed towards the medical device.

In some embodiments, the controlling method may further include: in response to determining that the mobile patient bed is moved to a track within the docking region of the medical device, controlling, by the track, the mobile patient bed to dock with the medical device.

In some embodiments, the controlling method may further include: in response to determining that the mobile patient bed moves to a preset depth of the medical device, obtaining docking completion information between the mobile patient bed and the medical device.

In some embodiments, the controlling method may further include: in response to determining that the mobile patient bed moves to the preset depth of the medical device, fixing the first docking structure on the mobile patient bed and the second docking structure on the medical device by initiating a retaining structure.

In some embodiments, the controlling method may further include: detecting, by a detection module, information related to the mobile patient bed, and alerting and/or correcting abnormality information of the detected information related to the mobile patient bed

One of the embodiments of the present disclosure provides a controlling system for docking a mobile patient bed with a medical device. The controlling system may include: a target positioning information obtaining module configured to obtain, based on a docking instruction, positioning information of a target; a patient bed movement control module configured to control, based on the positioning information of the target, a movement of the mobile patient bed; and a docking module configured to control the mobile patient bed to dock with the medical device under a guidance of at least two docking structures in response to determining that the mobile patient bed moves into a docking region of the medical device.

One of the embodiments of the present disclosure provides a controlling device for docking a mobile patient bed with a medical device. The device may include at least one processor and at least one memory; the at least one storage device being configured to store computer instructions; the at least one processor being configured to perform at least a portion of the computer instructions to implement a controlling method for docking a mobile patient bed with a medical device.

One of the embodiments of the present disclosure provides a non-transitory computer readable storage medium storing computer instructions, wherein when executing the computer instructions in the non-transitory computer readable storage medium, a computer implements a controlling method for docking a mobile patient bed with a medical device.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering indicates the same structure, wherein:
FIG. 1 is a schematic diagram illustrating an application scenario of a controlling system according to some embodiments of the present disclosure;
FIG. 2 is an exemplary flowchart illustrating a controlling method according to some embodiments of the present disclosure;
FIG. 3 is a schematic diagram illustrating a structure of a patient bed moving along an automatically planned path or a preset path according to some embodiments of the present disclosure;
FIG. 4 is a schematic diagram illustrating a structure of a patient bed performing a following movement based on positioning information of a mobile device according to some embodiments of the present disclosure;

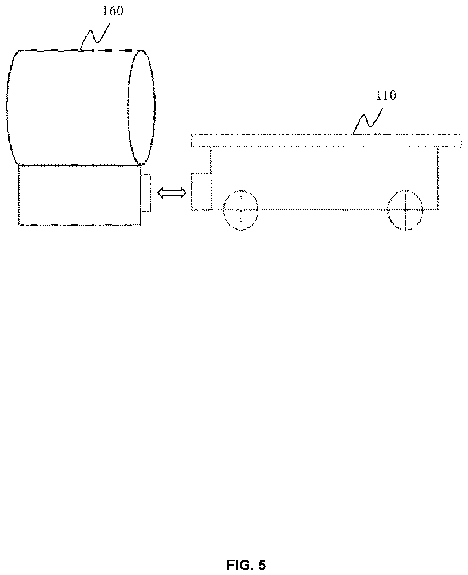

FIG. 5 is a schematic diagram illustrating a structure of a patient bed docking with a medical device according to some embodiments of the present disclosure;
FIG. 6 is a schematic diagram illustrating a structure of a controlling system according to some embodiments of the present disclosure; and
FIG. 7 is a schematic diagram illustrating a signal transmission between a patient bed movement control module and a mobile device control module according to some embodiments of the present disclosure.

### DETAILED DESCRIPTION

In order to more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for those skilled in the art to apply the present disclosure to other similar scenarios according to these drawings without creative labor.

It should be understood that the terms "system", "device", "unit," and/or "module" as used herein is a way to distinguish between different components, elements, parts, sections, or assemblies at different levels. However, these words may be replaced by other expressions if other words accomplish the same purpose.

As shown in the present disclosure and in the claims, unless the context clearly suggests an exception, the words "one," "a ", "an", and/or "the" do not refer specifically to the singular, but may also include the plural. Generally, the terms "including" and "comprising" suggest only the inclusion of clearly identified operations and elements that do not constitute an exclusive list, and the method or device may also include other operations or elements.

Flowcharts are used in the present disclosure to illustrate operations performed by a system according to embodiments of the present disclosure. It should be appreciated that the preceding or following operations are not necessarily performed in an exact sequence. Instead, operations may be processed in reverse order or simultaneously. Also, it may be possible to add other operations to these processes, or to remove an operation or operations from these processes.

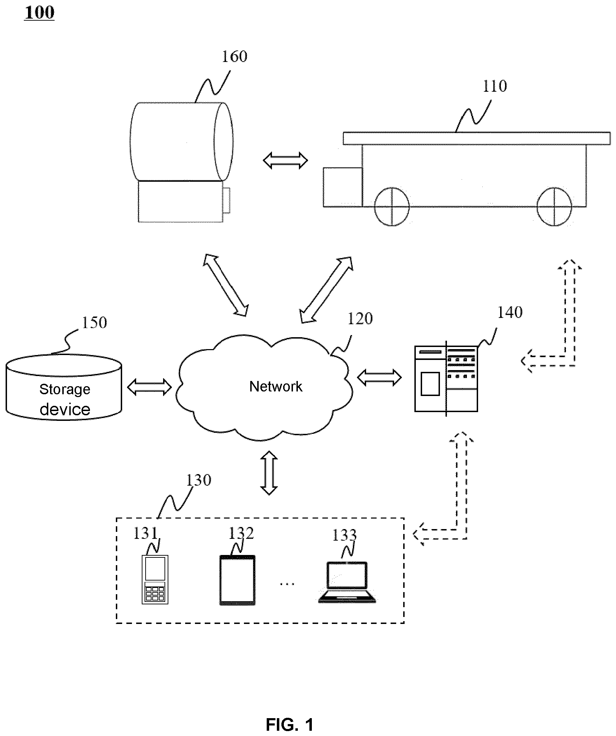

FIG. 1 is a schematic diagram illustrating an application scenario of a controlling system according to some embodiments of the present disclosure. A controlling system 100 for docking a mobile patient bed and a medical device may include a patient bed 110, a network 120, at least one terminal 130, a processing device 140, a storage device 150, and a medical device 160. Components of the system may be connected to each other through the network 120. For example, the patient bed 110 and the at least one terminal 130 may be connected or in communication through the network 120.

The patient bed 110 may include a mobile patient bed for transferring a patient. In some embodiments, the patient bed 110 may include a patient bed body, mobile wheels, a driving device, a signaling device, and a docking structure. The patient bed body may be configured to load the patient. The driving device may be configured to drive the moving wheels to rotate for a movement of the patient bed 110. The signaling device may be configured to send and/or receive information. The docking structure may be realized with a corresponding docking structure on the medical device 160.

The network 120 may include any suitable network capable of facilitating an exchange of information and/or data between the mobile patient bed and the controlling system 100 for the medical device. In some embodiments, at least one of the components of the controlling system 100 (e.g., the patient bed 110, the processing device 140, the storage device 150, the at least one terminal 130, the medical device 160) may exchange the information and/or data with at least one other component of the controlling system 100 through the network 120. For example, the processing device 140 may obtain positioning information and/or a movement parameter of the mobile patient bed 110 from the mobile patient bed 110 through the network 120. For another example, the processing device 140 may obtain an operator (e.g., a doctor) instruction from the at least one terminal 130 through the network 120. The network 120 may include a public network (e.g., the Internet), a private network (e.g., a local area network (LAN)), a wired network, a wireless network (e.g., an 802.11 network, a Wi-Fi network), a frame relay network, a virtual private network (VPNs), a satellite network, a telephone network, a router, a hub, a switch, a server computer, and/or any combination thereof. For example, the network 120 may include a wired network, a cable network, a fiber optic network, a telecommunication network, an intranet, a wireless local area network (WLAN), a metropolitan area network (MAN), a public switched telephone network ( PSTN), a Bluetooth^{™} network, a ZigBee^{™} network, a near field communication (NFC) network, etc., or any combination thereof. In some embodiments, the network 120 may include at least one network access point. For example, the network 120 may include a wired and/or wireless network access point, such as a base station and/or an Internet exchange point, and the at least one component of the controlling system 100 may be connected to the network 120 through the access point to exchange the data and/or information.

The at least one terminal 130 may be in communication and/or connection with the patient bed 110, the processing device 140, the storage device 150, and/or the medical device. For example, the at least one terminal 130 may obtain a docking instruction from the processing device 140. For another example, the at least one terminal 130 may obtain, through the patient bed 110, positioning information of the patient bed 110, and send the positioning information of the patient bed 110 to the processing device 140 for processing. In some embodiments, the at least one terminal 130 may include a mobile device 131, a tablet computer 132, a laptop computer 133, etc., or any combination thereof. For example, the mobile device 131 may include a cell phone, a personal digital assistant (PDA), a mobile bracelet, a handheld remote control, etc., or any combination thereof. In some embodiments, the at least one terminal 130 may include an input device, an output device, etc. The input device may include letters, numbers, and other keys. The input device may optionally be a keyboard input, a touch screen (e.g., with haptic or tactile feedback) input, a voice input, an eye-tracking input, a brain monitoring system input, or any other similar input mechanism. Input information received through the input device may be transmitted to the processing device 140 for further processing. Other types of input devices may include a cursor control device, such as a mouse, a trackball, or cursor arrow keys, etc. The output device may include a monitor, a speaker, a printer, etc. or any combination thereof. In some embodiments, the at least one terminal 130 may be a portion of the processing device 140.

The processing device 140 may process the data and/or information obtained from the patient bed 110, the storage device 150, the at least one terminal 130, the medical device 160, or other components of the controlling system 100 for docking a mobile patient bed with a medical device. For example, the processing device 140 may determine target positioning information of the medical device 160 based on a positioning signal from a signal transmission device of the medical device 160. For another example, the processing device 140 may obtain an automatically planned path based on the positioning information of the patient bed 110 and the medical device 160, and control, through a movement control device of the patient bed 110, the patient bed 110 to move along the automatically planned path. In some embodiments, the processing device 140 may be a single server or a server group. The server group may be centralized or distributed. In some embodiments, the processing device 140 may be local or remote. For example, the processing device 140 may access the information and/or data through the network 120 from the patient bed 110, the storage device 150, the medical device 160, and/or the at least one terminal 130. For another example, the processing device 140 may be directly connected to the patient bed 110, the storage device 150, the medical device 160, and/or the storage device 150 to access the information and/or data. In some embodiments, the processing device 140 may be implemented on a cloud platform. For example, the cloud platform may include a private cloud, a public cloud, a hybrid cloud, a community cloud, a distributed cloud, an inter-cloud cloud, a multi-cloud, etc., or any combination thereof.

The storage device 150 may store data, instructions, and/or any other information (e.g., the positioning information of the patient bed 110, a movement path of the patient bed 110, etc.). In some embodiments, the storage device 150 may store the data obtained from the patient bed 110, the medical device 160, the at least one terminal 130, and/or the processing device 140. In some embodiments, the storage device 150 may store the data and/or instruction used by the processing device 140 to execute or use to accomplish the exemplary methods of the present disclosure. In some embodiments, the storage device 150 may include a mass memory, a removable memory, a volatile read-write memory, a read-only memory (ROM), etc., or any combination thereof. In some embodiments, the storage device 150 may be implemented on the cloud platform.

In some embodiments, the storage device 150 may be connected to the network 120 to communicate with at least one other component in the controlling system 100 (e.g., the processing device 140, the at least one terminal 130). The at least one component of the controlling system 100 may access the data or instructions stored in the storage device 150 through the network 120. In some embodiments, the storage device 150 may be a portion of the processing device 140.

The medical device 160 may include an imaging device, an analyzing device, etc. For example, the medical device 160 may be a digital radiography (DR) device, a computed radiography (CR), a digital fluorography (DF), a positron emission tomography (PET), a cone-beam computed tomography (CBCT), a single photon emission computed tomography (SPECT), an image-guided radiation therapy device, a biochemical immunoassay analyzer, a CT scanner, a magnetic resonance scanner, an electrocardiograph, a B-ultrasound, etc. In some embodiments, the medical device 160 may include a rack, a docking structure, a detector, a detection area, a patient bed, and a ray generator. The rack may be configured to support the detector and the ray generator. The docking structure can be connected to a corresponding docking structure on the patient bed 110. A scanning object may be placed on the patient bed for scanning. The scanning object may include a patient, a molded body, or other object being scanned. The patient bed may be parallel to a floor. The ray generator may emit X-rays to the scanning object. By scanning the scanning object, the medical device 160 may obtain scanning data to generate (or reconstruct) an image.

It should be noted that the foregoing description is provided for illustrative purposes only and is not intended to limit the scope of the present disclosure. For those skilled in the art, a wide variety of changes and modifications may be made under the guidance of the contents of the present disclosure. Features, structures, methods, and other characteristics of the exemplary embodiments described in the present disclosure may be combined in various ways to obtain additional and/or alternative exemplary embodiments. For example, the storage device 150 may be a data storage device that includes a cloud computing platform, such as a public cloud, a private cloud, a community, and a hybrid cloud, etc. However, these changes and modifications do not depart from the scope of the present disclosure.

In some embodiments, the movement of the patient bed 110 may be mainly manually controlled, i.e., an operator may push the patient bed 110. For example, when a patient needs to be transported to various scanning rooms for radiographs, MRI scans, and/or radiographs, etc., current mobile patient beds are mainly manually pushed, i.e., an operator (e.g., a healthcare worker) may manually push the patient bed to move the patient bed to the scanning rooms, and then the patient bed 110 may be docked with the medical device 160. In the process of pushing the patient bed 110, the patient bed 110 may have to pass through slopes, stages, elevators, potholes, and other surfaces, and may encounter corners, etc., and a duration of the entire pushing process may be long (e.g., which spends 10 to 15 minutes). Therefore, much effort is required from the operator. In the process of docking the patient bed 110 with the medical device 160, there may be a high requirement on a docking position. The patient bed 110 may be heavy, making the docking inconvenient, and the operator needs to spend much time and energy in the docking process. In this case, the automatic control of the movement of the patient bed and the automatic docking may be particularly important. In some embodiments, the patient bed 110 may be automatically controlled to move to the docking region where the medical device 160 is located, and guided by the docking structure, the docking of the patient bed 110 with the medical device 160 may be performed automatically. This automated docking technique may be applicable to the mobile patient beds and the medical devices that is able to be docked therewith, as will be elaborated in the embodiments of the present disclosure.

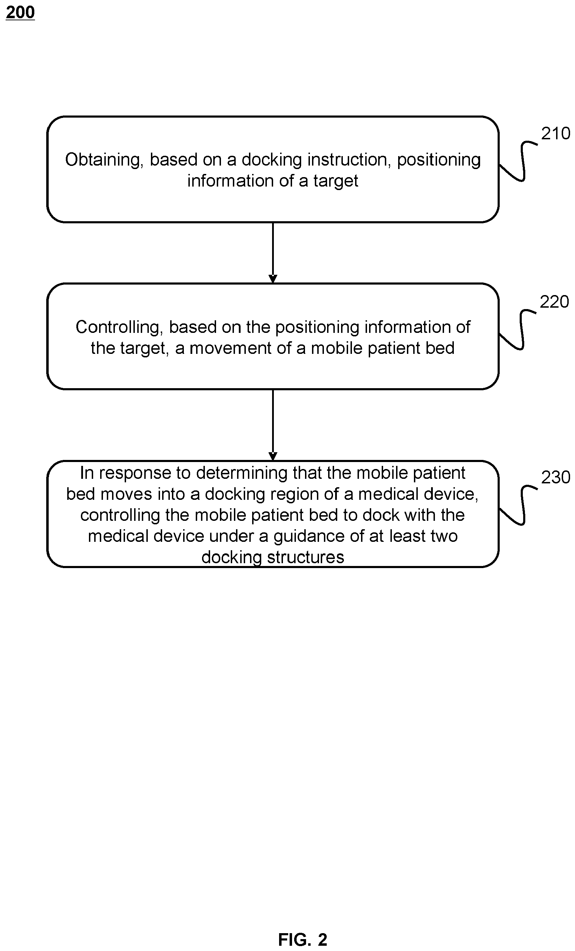

FIG. 2 is an exemplary flowchart illustrating a controlling method according to some embodiments of the present disclosure.

As shown in FIG. 2, some embodiments of the present disclosure provide a controlling method for docking a mobile patient bed with a medical device, and a process 200 of the controlling method may include following operations. In some embodiments, the process 200 may be performed by a terminal (e.g., the terminal 130).

In 210, positioning information of a target is obtained based on a docking instruction. In some embodiments, the operation 210 may be performed by a target position obtaining module 610.

In some embodiments, the docking instruction may be request information for transferring the patient bed 110 to the designated medical device 160 for docking. In some embodiments, when the patient needs to be transferred to the medical device 160 for examination, an operator (e.g., a medical worker) may input the docking instruction to the processing device 140. The processing device 140 may obtain the request information of the docking instruction and then proceeds to the next operation of analyzing and processing.

In an actual use in a hospital, there may be a plurality of the medical devices 160 of a plurality of different types distributed among different scanning rooms. There may also be a plurality of patient beds 110 distributed on different floors and in different wards. In some embodiments, each medical device 160 may have a device code. The device code may include information such as a type, a specification of the device and a department to which the device belongs. In some embodiments, each patient bed 110 may have a patient bed code. The patient bed code may include the floor on which the patient bed is located, a ward number of the ward in which the patient bed is located, and a position of the patient bed.

In some embodiments, when the docking instruction is input, the patient bed code for the patient bed 110 and a device code for the medical device 160 may be input. Based on the input docking instruction, the positioning information of the patient bed 110 and the positioning information of the medical device 160 may be obtained. In some embodiments, the docking instruction may be input by the operator through the mobile device 131 (e.g., a cell phone, a remote control, etc.), and based on the input docking instruction, the positioning information of the mobile device 131 may also be obtained.

The positioning information of the target refers to positioning information of a datum that guides the movement of the patient bed 110. The datum refers to a reference object that guides the movement of the patient bed 110. In some embodiments, the target may be a medical device (e.g., the medical device 160), which is stationary and parked within the scanning room, and may be the datum for the movement of the patient bed 110. The patient bed 110 may move towards the medical device 160, as detailed below. In some embodiments, the target may be the mobile device 131 (e.g., the mobile phone, the remote control, etc.). For example, the moving mobile device 131 may be the datum for the movement of the patient bed 110, and the patient bed 110 may follow the mobile device 131 to move, as detailed below.

In some embodiments, the positioning information may include position information etc. In some embodiments, the positioning information may also include the position information at a certain time point. For example, the positioning information may include a specific position (e.g., latitude/longitude coordinates where the position is located) where the target is located at a certain time point. Each moment in time may correspond to a position of a target. For another example, the positioning information may include a current location of the target. The positioning information may be obtained based on a positioning technique. Exemplary positioning techniques may include a GPS positioning technique, a WIFI positioning technique, an ultra-wideband positioning technique, a Bluetooth positioning technique, an infrared positioning technique, an ultrasonic positioning technique, a ZigBee positioning technique, a radio frequency recognition positioning technique, a light tracking positioning technique, an image analysis technique, a beacon positioning technique, a computer vision positioning technique, etc. In some embodiments, the processing device 140 may obtain the positioning information of the target periodically or in real time. For example, the processing device 140 may obtain the positioning information of a signal transmission device at fixed length intervals such as 500 milliseconds, 1 second, 2 seconds, 3 seconds, 5 seconds, etc. In some embodiments, the processing device 140 may obtain the positioning information of the target at a preset time point. For example, a fixed time point for obtaining the positioning information may be preset, such as setting the two time points of 16:10:50 and 16:11:00 as the preset time points.

In 220, a movement of the mobile patient bed is controlled based on the positioning information of the target. In some embodiments, operation 220 may be performed by a patient bed movement control module 620.

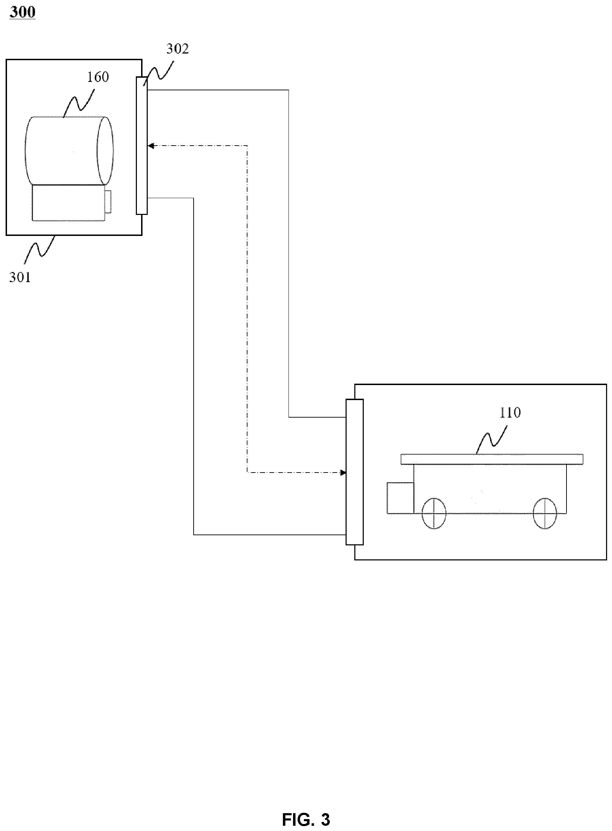

In some embodiments, the positioning information of the target may include the positioning information of the medical device. In this embodiment, a stationary medical device 160 may be used as a datum for the movement of the patient bed 110, and the patient bed 110 may move along a certain path toward the medical device 160. FIG. 3 is a schematic diagram illustrating a structure of a patient bed moving along an automatically planned path or a preset path according to some embodiments of the present disclosure.

In some embodiments, the processing device 140 may obtain an automatically planned path based on the positioning information of the medical device 160 to control the movement of the patient bed 110. The automatically planned path may indicate a route or trajectory of the patient bed 110 from the current position to the target position (i.e., the position of the processing device 140).

In some embodiments, based on the docking instruction, the processing device 140 may obtain the positioning information of the patient bed 110 and the positioning information of the medical device 160, and obtain the automatically planned path based on the position between the patient bed 110 and the medical device 160, and a spatial layout of the hospital by calculation. The processing device 140 may send the automatically planning path to the patient bed 110 and control the driving device of the patient bed 110, so as to control the patient bed 110 to move along the automatically planning path into the docking region of the medical device 160.

In some embodiments, during the movement of the patient bed 110, a signaling device on the patient bed 110 may send real-time positioning information of the patient bed 110 to the processing device 140. The processing device 140 may recalculate and update the automatically planned path based on the obtained real-time information of the patient bed 110. For example, the automatically planned path needs to be updated when the patient bed 110 encounters traffic information that affects its movement.

In some embodiments, one or more preset paths from each patient bed 110 to each medical device 160 may be stored in the storage device 150. The preset paths may be preset and stored in the storage device 150 by the processing device 140 based on the positioning information of each patient bed 110 and the positioning information of each medical device 160. Based on the docking instruction, the processing device 140 may directly retrieve the preset path in the storage device 150 and control the movement of the patient bed 110 along the preset path. For example, when an operator (e.g., a healthcare worker) enters a docking instruction, the processing device 160 may retrieve, based on coding information of the patient bed 110 and the coding information of the medical device 160 specified in the docking instruction, a suitable preset path from the storage device 150, send the suitable preset path to the patient bed 110, and control the driving device of the patient bed 110 to control the movement of the patient bed 110 along the preset path to within the docking region of the medical device 160.

In some embodiments, when the medical patient bed 110 encounters the traffic information that affects its movement, the processing device 140 may re-retrieve a reasonable planned path based on the current positioning information of the medical patient bed 110 to ensure that the medical patient bed 110 is able to effectively and quickly arrive the docking region of the medical device 160.

In some embodiments, the processing device 140 may also obtain the traffic information around the patient bed 110. The processing device 140 may update the automatically planned path for the patient bed 110 based on the traffic information. The traffic information may include obstacle information, path information, etc., or a combination thereof, in a direction of movement of the patient bed 110.

In some embodiments, the patient bed 110 may be disposed with one or more cameras, and the processing device 140 may obtain an optical image around the patient bed 110 through the cameras. The processing device 140 may analyze the obtained optical image to recognize the traffic information around the patient bed 110, and then update the automatically planned path through a path planning module. In some embodiments, the processing device 140 may also obtain the traffic information through an infrared recognition technology, a laser recognition technology, an ultrasonic recognition technology, etc., and the manner of obtaining the traffic information is not limited herein.

The obstacle information may include a presence of an obstacle, a category of the obstacle (e.g., a person, a wall, a patient bed, a doorsill, etc.), a height of the obstacle (e.g., 3 meters, 1 meter, 0.5 meters, 0.2 meters, 0.05 meters), etc., and any combination thereof. In some embodiments, when determining that an obstacle exists in the direction of movement of the patient bed 110, the processing device 140 may retrieve an obstacle avoidance path from the storage device 150 based on the height and/or the category of the obstacle. The processing device 140 may update the path of movement of the patient bed 110 to the obstacle avoidance path retrieved. For example, when the height of the obstacle is higher than a certain value (e.g., 0.1 meters), the processing device 140 may retrieve the obstacle avoidance path that bypasses the obstacle. For another example, when the height of the obstacle is lower than the certain value (i.e., 0.1 meters), the processing device 140 may not update the path of movement of the patient bed 110. At this time, the processing device 140 may increase a driving torque applied to the patient bed 110. For another example, when the obstacle is determined to be a person, the patient bed 110 may stop and wait for the obstacle (i.e., the person) in front of the patient bed 110 to disappear before moving towards the target location. The obstacle avoidance path may also include more forms, which are not described herein.

The path information may include a width of the path, a category of the path (e.g., a curve), etc. In some embodiments, the processing device 140 may recognize the width of the path in front of the patient bed 110. When determining that the width of the path is less than a certain value, the processing device 140 may re-plan the path of movement and/or control the patient bed 110 to sound an alarm alert. For example, when the width of the path is less than a width of the patient bed 110, the patient bed 110 may stop and sound the alarm alert. In some embodiments, when it is determined that the curve is ahead (e.g., an intersection or a T-intersection), the processing device 140 may control the patient bed 110 to sound a turning alert alarm, thereby prompting the operator (or other pedestrians) to watch out for the patient bed 110 to avoid bumping into a wall or to keep clear.

In some embodiments, the patient bed 110 may be configured with one or more sensors, and the processing device 140 may sense, through the sensors, the traffic information where the patient bed 110 is located, and the processing device 140 may analyze the traffic information to recognize the traffic information around the patient bed 110. In some embodiments, the one or more sensors may include infrared sensors, laser sensors, ultrasonic sensors, etc., or a combination thereof.

In some embodiments, the positioning information of the target may include the positioning information of the mobile device 131. The embodiment uses the mobile device 131 as the target (i.e., the datum) for the movement of the patient bed 110. In some embodiments, the mobile device 131 may be one of the terminals 130, e.g., a cell phone, a remote control, etc.

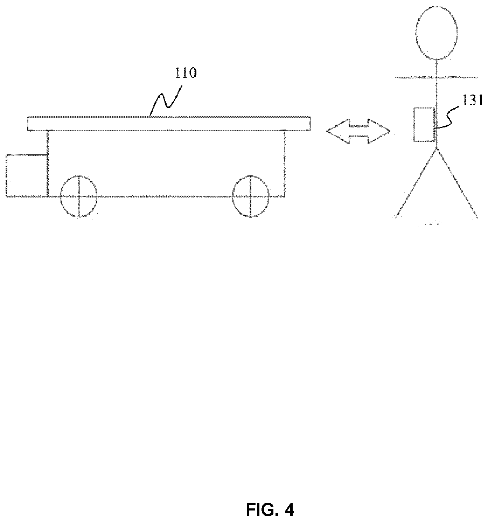

In some embodiments, the patient bed may be controlled to follow the mobile device 131 based on the positioning information of the mobile device 131. FIG. 4 is a schematic diagram illustrating a structure of a patient bed performing a following movement based on positioning information of a mobile device. The mobile device 131 may be carried by the operator (e.g., the healthcare worker) to perform the following movement with a movement of the operator, thereby controlling the movement of the patient bed 110. For example, the patient bed 110 may automatically follow the movement of the mobile device 131 based on the received positioning information of the mobile device 131 (referred to as a "follow mode"). The following refers to that the patient bed 110 moves with the movement of the mobile device 131. In some embodiments, when the patient bed 110 follows the movement of the mobile device 131, a speed of the movement of the patient bed 110 (i.e., a following speed) may be equal to the speed of the movement of the mobile device 131, and a direction of movement of the patient bed 110 may be the same as a direction of movement of the mobile device 131, and the mobile device 131 and the patient bed 110 may move forward synchronously. In some embodiments, the speed of movement of the patient bed 110 may also be unequal to the speed of movement of the mobile device 131 during a process of following of the patient bed 110. For example, the speed of movement of the patient bed 110 may be less than the speed of movement of the mobile device 131, with a speed difference remains constant. For another example, the patient bed 110 may move towards the mobile device 131 (or the operator) at a preset speed (e.g., a default speed of the controlling system 100) based on the received positioning information of the mobile device 131.

In some embodiments, the preset speed may be set with a plurality of different movement speeds, and one of the preset speeds may be adaptively adjusted or selected in use. In some embodiments, the preset speed may be adaptively adjusted or selected based on whether or not a patient is being carried on the patient bed 110 (e.g., as determined by bed board load-bearing information). For example, a greater speed of movement may be adaptively adjusted or selected when no patient is detected to be loaded on the patient bed 110, and when a patient is detected to be loaded on the patient bed 110, the movement speed may be adaptively adjusted or selected to be slower, thereby maintaining a movement stability of the patient bed 110 to ensure a safety of the patient carried on the patient bed 110. In some embodiments, the preset speed may also be set with a plurality of different movement speeds based on different spatial regions (e.g., a ward, a corridor, an elevator room, etc.), and the processing device 140 may self-adaptively adjust or select the preset speed based on determining that the patient bed 110 is located in a different spatial region. For example, a slower speed of movement may be adaptively adjusted or selected when the patient bed 110 is detected to be in the ward, a greater speed of movement may be adaptively adjusted or selected when the patient bed 110 is detected to be in an empty corridor, and when the patient bed 110 is detected to be in the elevator room, the speed of movement may be adaptively adjusted or selected to be 0. It is worth noting that, in a process of the patient bed 110 following the movement of the mobile device 131, the present disclosure does not have limit on a relative orientation of the patient bed 110 to the mobile device 131. For example, the patient bed 110 may be located in front, behind, to the left, to the right, etc. of the mobile device 131. In some embodiments, an on/off button may be disposed on the mobile device 131. The operator may press the on/off button to control the mobile device 131 to emit the positioning information. The mobile device 131 may send the positioning information to the processing device 140 after the operator presses the on/off button.

In some embodiments, when the patient bed 110 follows the movement of the mobile device 131, a relative position of the patient bed 110 and the mobile device 131 may be changed, but a relative distance between the patient bed 110 and the mobile device 131 may always be maintained within a preset distance range (for example, the preset distance range may be 0.1 to 1 meter). For example, in a scenario of turning, the relative positions of the patient bed 110 and the mobile device 131 may change before and after the turning, and during the turning process, as a traveling speed of the patient bed 110 slows down as a result of the turning action, the relative distance between the patient bed 110 and the mobile device 131 may change, but may always be maintained within the preset distance range. In some embodiments, when the relative distance between the patient bed 110 and the mobile device 131 exceeds the preset distance range, the mobile device 131 may stop moving and wait for the patient bed 110 to move towards the mobile device 131 until the relative distance between the two is within the preset distance range, then the mobile device 131 may start moving again.

In some embodiments, the docking instruction may be input through the mobile device 131. The patient bed 110 may obtain the positioning information of the mobile device 131 based on the docking instruction. The docking instruction obtained by the patient bed 110 may be obtained through the on/off button of the mobile device 131 as described above, or by other means. For example, the patient bed 110 itself may be provided with a control button, and when the control button is triggered, the patient bed 110 may obtain the docking instruction, and search for the target (e.g., by infrared, Bluetooth, etc., to search for a nearby mobile device 131) and determine the positioning information of the target (i.e., the positioning information of the mobile device 131). For another example, the patient bed 110 may also obtain the docking instruction from other terminals, these terminals being terminals other than the following target (e.g., a computer in a doctor's office), and the other terminals may also give instructions to the patient bed by on/off buttons, voice control, etc. to send the docking instruction to the patient bed 110. In some embodiments, the target may be understood to be a following object of the patient bed 110; or may also be understood to be the signaling device (a signal transmission device or a signal receiving device) carried by the following object. The target herein may be the mobile device 131 described above if the patient bed 110 obtains the positioning information through the signaling device carried by the following object.

In some embodiments, the processing device 140 may determine a distance between the mobile device 131 and the patient bed 110, and determine that when the distance between the mobile device 131 and the patient bed 110 is greater than a signal response distance (e.g., 5 meters, 4 meters, 3 meters, etc.), the processing device 140 may not obtain the positioning information of the mobile device 131. At this time, the mobile device 131 may fail to make a connection with the patient bed 110, and may not be able to control the movement of the patient bed 110. In other words, the mobile device 131 may control the movement of the patient bed 110 only when the distance between the mobile device 131 and the patient bed 110 is within the signal response distance. In some embodiments, the distance between the mobile device 131 and the patient bed 110 may be calculated based on the position of the mobile device 131 and the position of the patient bed 110. For example, the processing device 140 may obtain the position of the mobile device 131 and the position of the patient bed 110, and then obtain the distance between the mobile device 131 and the patient bed 110 by calculation. In some embodiments, the distance between the mobile device 131 and the patient bed 110 may be obtained directly based on a distance sensor. An exemplary distance sensor may include an ultrasonic ranging sensor, a laser ranging sensor, an infrared ranging sensor, a radar sensor, etc.

In some embodiments, the processing device 140 may also obtain a movement parameter of the mobile device 131 based on the positioning information of the mobile device 131, as well as the positioning information of the patient bed 110. The movement parameter is to be determined as a following movement parameter of the patient bed 110.

In some embodiments, the movement parameter may include a movement distance, a movement direction, a movement time, a movement speed, etc. The following movement parameter may include a following movement distance, a following movement direction, a following movement time, a following movement speed, etc. For example, the processing device 140 may determine that a distance between a first position and a second position is the movement distance of the mobile device 131, i.e., the processing device 140 may determine that the distance between the first position and the second position is the following movement distance of the patient bed 110. The first position and the second position may refer to two different specific positions before and after the movement of the patient bed 110; the first position may also refer to a position of the patient bed 110 at a certain time point, and the second position may be a position of the patient bed 110 after moving for a certain duration (e.g., 1 minute, 2 minutes, 3 minutes, etc.) from the first position. For example, the processing device 140 may determine that a direction pointing from the first position to the second position is the direction of movement of the mobile device 131, i.e., the processing device 140 may determine that the direction between the first position and the second position is the direction of the following movement of the patient bed 110. For another example, the processing device 140 may determine that a time from the first position to the second position is a time of movement of the mobile device 131, i.e., the processing device 140 may determine that the direction from the first position to the second position is a direction of the following movement of the patient bed 110. For another example, the device 140 may obtain the following movement speed of the patient bed 110 from the driving device of the patient bed 110. The following movement parameter may also include other movement-related parameters, such as an estimated time of arrival, etc., which are not limited herein.

In some embodiments, the mobile device 131 may be a remote control for controlling the movement of the patient bed 110 (referred to as a "remote control mode"). The operator may directly manipulate the driving device of the patient bed 110 through the mobile device 131 to control the travel of the patient bed 110. For example, the mobile device 131 may be provided with various buttons such as front, back, left, right, start, stop, accelerate, decelerate, etc., and the operator may manipulate the patient bed 110 by pressing the various buttons to control the travel of the patient bed 110 toward the medical device 160. In some embodiments, the mobile device 131 may also be a touch device (e.g., a smart bracelet, a smart phone, a smart tablet, etc.) configured to control the movement of the patient bed 110.

In some embodiments, a movement mode of the patient bed 110 may include a following mode and a remote control mode, and the movement mode may be automatically switched according to different spatial regions in which the patient bed 110 is located. In practical application scenarios (e.g., in the hospital), the patient bed 110 generally needs to pass through the ward, the corridor, the elevator room, and a scanning room to travel from the ward to the scanning room. In some embodiments, the processing device 140 may select the movement mode based on the detected region of space in which the patient bed 110. For example, when the patient bed 110 is detected to be in the corridor, the patient bed 110 may be automatically switched to the following mode; or for example, when the patient bed 110 is detected to be in the ward, the elevator room, or the scanning room, the patient bed 110 may be automatically switched to the remote control mode. In some embodiments, due to a small space in the elevator room and the scanning room, it may be preset that when the patient bed 110 is detected to be in the elevator room or the scanning room, the following mode of the patient bed 110 may be immediately switched from the following mode to the remote control mode.

In some embodiments, when the patient bed 110 is outside of a guiding region, the processing device 140 may control the patient bed 110 to perform the following movement based on the positioning information of the mobile device 131 (see embodiments above). When the patient bed 110 moves within the guiding region, the processing device 140 may stop the following movement of the patient bed 110 and switch to an intelligent guidance mode. The guiding region refers to a region contained within the distance between the patient bed 110 and the medical device 160 that satisfies an intelligent guiding preset distance. The intelligent guiding preset distance may be preset by the processing device.

In some embodiments, the intelligent guidance mode may include: according to the positioning information of the medical device 160, obtaining an automatically planned path of the patient bed 110 by calculation, and controlling the movement of the patient bed 110 along the automatically planned path until the patient bed 110 enters the docking region of the medical device 160. In some embodiments, the intelligent guidance mode may further include: controlling, by the processing device 140, based on the positioning information of the medical device 160 and the preset path extracted from the storage device 150, the patient bed 110 to move along the preset path until the patient bed 110 enters the docking region of the medical device 160.

In some embodiments, the scanning room 301 may be disposed within the guiding region. In some embodiments, as shown in FIG. 3, an in-place sensor 302 may be provided at the doorway of the scanning room 301 for sensing whether the patient bed 110 has reached the doorway of the scanning room 301 (i.e., for determining whether the patient bed 110 is within the guiding region). The in-place sensor 302 may be a sensor mounted on the door frame of the scanning booth 301, such as on the top, the bottom, the left, the right, of the door, or any combination thereof. The in-place sensor 302 may include an infrared sensor, a laser sensor, an ultrasonic sensor, etc., and the manner of sensing an arrival of the patient bed 110 at the scanning room 301 is not limited herein.

In some embodiments, it may be set that when the distance between the patient bed 110 and the medical device 160 satisfies the intelligent guiding preset distance, the patient bed 110 may be determined to be in the guiding region (e.g., the intelligent guiding preset distance may be set to 0.5m, 1 m, 1.5m, etc.). In some embodiments, when the processing device 140 determines, based on the positioning information of the patient bed 110, that the distance between the patient bed 110 and the medical device 160 satisfies the intelligent guiding preset distance, the patient bed 110 may be switched from the following mode to the intelligent guiding mode automatically.

In some embodiments, the in-place sensor may also be disposed on the patient bed 110 or on the medical device 160 for sensing whether the distance between the patient bed 110 and the medical device 160 is less than the intelligent guiding preset distance (i.e., for determining whether the patient bed 110 is within the guiding region).

In some embodiments, when the patient bed 110 moves within the guiding region, the processing device 140 may send a confirmation message/prompt message for switching the movement mode of the patient bed 110 (e.g., from a following mode to an intelligent guiding mode) to the mobile device 131, and the operator (e.g., a doctor) may operate according to the message on the mobile device 131 to complete the switching of the movement mode of the patient bed 110.

In some embodiments, when the patient bed 110 is outside the guiding region, the processing device 140 may control the patient bed 110 to perform the following movement based on the positioning information of the mobile device 131 (see the above-described embodiment); and when the patient bed 110 is moved into the guiding region, the positioning information of the patient bed 110 may be obtained by a wireless positioning module, and the processing device 140 may control the movement of the patient bed 110 based on the positioning information of the patient bed 110 until the patient bed 110 runs into the docking region of the medical device 160.

In some embodiments, the wireless positioning module may obtain the positioning information of the patient bed 110 through a wireless detection technology.

The mode of positioning of the wireless positioning module may be directly or indirectly determining positioning parameters like a time, a phase difference, an amplitude, or a frequency variation during a propagation of radio signals between a fixed point (e.g., the medical device 160) of a known position and the patient bed, thereby determining the position of the point to be fixed (e.g., the patient bed 110) using a position line.

In some embodiments, the wireless positioning module may be one or more of a 2.4G wireless transmission positioning module, an infrared positioning module, a millimeter wave positioning module, a pulsed radiolocation module, a phased radiolocation module, a pulsed one phased radiolocation module, etc.

In some embodiments, when the patient bed 110 is within the scanning room 301 but is not yet within the guiding region, the patient bed 110 may still be controlled to perform the following movement based on the positioning information of the mobile device 131 until the patient bed 110 enters the docking region of the medical device 160.

In some embodiments, a display device (e.g., a display screen) may be provided on the patient bed 110, and the display device may display movement information (e.g., a current position, a movement path, etc.) of the patient bed 110, which helps the operator (e.g., a healthcare worker) to observe the movement information of the patient bed 110 in real time. In some embodiments, the display device may also display an automatically planned path and the current position of the movement of the patient bed calculated by the processing device 140 based on the positioning information of the medical device 160. In some embodiments, the display device may also display the preset path and the current movement position of the patient bed in the storage device 150.

In some embodiments, a display mode of the display device may include, but not limited to, an image display, a voice announcement, and/or any combination thereof. For example, the display device (e.g., the display screen) may be provided on the mobile device 131 (e.g., the cell phone, the remote control, etc.), and the operator (e.g., the healthcare worker), by operating the mobile device 131, may send the docking instruction to the processing device 140. The processing device 140 may send the automatically planned path (or the preset path) to the mobile device 131 based on the positioning information of the mobile device 131 (or the patient bed 110) and the positioning information of the medical device 160, and may intuitively display the information by image through the display device, which is easy for the operator to observe. In some embodiments, the display device may also display the movement path of the patient bed 110 when the patient bed 110 performs the following movement. In some embodiments, the display device may also display the traffic information around the patient bed 110. In some embodiments, the display device may also display the movement parameters, etc., of the patient bed 110.

In some embodiments, the display device may also be provided on other terminals 130 (e.g., the cell phones, the computers, etc.).

In 230, in response to determining that the patient bed 110 moves into the docking region of the medical device 160, the patient bed 110 is controlled to dock with the medical device 160 under a guidance of at least two docking structures. In some embodiments, the operation 230 may be performed by a docking module 630.

In some embodiments, the docking region may be a docking response range in which the patient bed 110 and the medical device 160 may perform a docking action. For example, the docking may start when the patient bed 110 moves to a certain docking response range from the medical device 160. Then the above docking response range may be the docking region of the patient bed 110 and the medical device 160.

In some embodiments, the docking structures may include a docking structure on the patient bed 110 and a docking structure on the medical device 160. The docking structure on the patient bed 110 and the docking structure on the medical device 160 may enable physical structural connections between the two, as well as electrical connections, and signaling connections.

In some embodiments, the controlling method for docking the mobile patient bed with the medical device may further include: when the patient bed 110 moves into the docking region of the medical device 160, the docking structure on the patient bed 110 starts to dock with the docking structure on the medical device 160, the docking structure on the medical device 160 may be capable of actively pulling the patient bed 110 toward the medical device 160 through the docking structure on the patient bed 110.

In some embodiments, the docking structure of the medical device 160 may apply a traction force on the docking structure of the patient bed 110, and under the action of the traction force, the patient bed 110 may passively move towards the medical device 160 to realize the docking of the patient bed 110 with the medical device.

In some embodiments, the connection of the docking structure of the patient bed 110 and the medical device 160 may include, but not limited to, a snap connection, a screw connection, etc. For example, if the docking structure of the medical device 160 is a snap, and the docking structure of the patient bed 110 may be a closed loop that mates with the snap, then when the snap of the patient bed 110 is connected to the closed loop of the medical device 160, the docking of the patient bed 110 and the medical device 160 may be realized. For example, if the docking structure of the patient bed 110 is a screw with external threads, and the docking structure of the medical device 160 is a threaded hole with internal threads that matches with the screw, then when the screw of the patient bed 110 is coupled with the threaded hole of the medical device 160, the docking of the patient bed 110 and the medical device 160 may be realized. The connection of the docking structure of the patient bed 110 and the medical device 160 may also be in other forms, such as a magnetic suction, a slide slot, and other forms of connection, which are not limited herein.

In some embodiments, the docking structure may also play a guiding role, i.e., when the docking structure of the patient bed 110 and the docking structure of the medical device 160 start to dock, the docking structures may achieve positioning and guiding functions in order to improve an accuracy of the docking. For example, the docking structure of the patient bed 110 and the medical device 160 may be screw-connected, and when the screw at a front end portion of the patient bed 110 and the threaded hole of the medical device 160 begin to dock, the threaded hole of the medical device 160 may be actively rotate along a center axis to achieve an active screw connection with the screw in the front end portion of the patient bed 110, and during the screw connection process, the patient bed 110 may continue to proceed in a direction of the medical device 160 under the action of the screw force until the screw reaches a certain depth to make the patient bed 110 and the medical device 160 dock successfully.

In some embodiments, the docking structure of the patient bed 110 and the medical device 160 may also be a magnetic positioning pin assembly. The magnetic positioning pin assembly may include a magnetic positioning pin and a magnetic pin hole. For example, the front end of the patient bed 110 may be disposed with a magnetic positioning pin, and when the patient bed 110 proceeds to the docking region of the medical device 160, the magnetic positioning pin at the front end portion of the patient bed 110 and the magnetic pin hole of the medical device 160 may begin to dock under the action of a magnetic force. The patient bed 110 may continue to proceed in the direction of the medical device 160 under the action of the magnetic force. A cooperation between the magnetic positioning pin and the magnetic pin hole may have a guiding effect on the movement of the patient bed 110 until the magnetic positioning pin reaches a certain depth to make the patient bed 110 and the medical device 160 dock successfully. In some embodiments, the docking structures of the patient bed 110 and the medical device 160 may include a plurality of groups of magnetic positioning pin assemblies, which may be two, three, four, or more groups to increase the guiding force and the magnetic force.

In some embodiments, the docking structures of the patient bed 110 and the medical device 160 may also be other forms of mechanical traction connection structures, which are not described herein.

In some embodiments, the docking region of the medical device 160 may be provided with rails that guides the patient bed 110. Then the controlling method of the mobile patient bed with the medical device may further include: when the patient bed 110 moves to the track within the docking region of the medical device 160, the patient bed 110 may be guided through the track to dock with the medical device 160.

In some embodiments, the track within the docking region may be disposed on the medical device 160, and the patient bed 110 may be provided with a slider that is compatible with the track, and during the docking of the patient bed 110 and the medical device 160, the slider may slide within the track to guide the patient bed.

In some embodiments, the track within the docking region may also be located on the floor of the scanning room. For example, two front wheels of the patient bed 110 may travel onto the track and into the docking region. In some embodiments, the patient bed 110 may continue to travel along the track controlled by the driving device of the patient bed 110 toward the medical device 160, with the track guiding the patient bed 110 until the patient bed 110 is successfully docked with the medical device 160. In some embodiments, the track may also provide the traction to the patient bed 110. For example, the track may be a moving track. After the two front wheels of the patient bed 110 have traveled to the track, the driving device of the patient bed 110 may stop, and the patient bed 110 may be driven by the moving track to travel towards the medical device 160. The moving track may act as both a guide and a traction for the patient bed 110.

In some embodiments, the controlling docking method for the mobile patient bed with the medical device may further include: when the patient bed 110 is docked and moved to a preset depth of the medical device 160, the processing device 140 may obtain docking completion information between the patient bed 110 and the medical device 160. The docking completion information may include a docking success and a docking failure.

The preset depth may be a distance traveled by the patient bed 110 in the direction of the medical device 160 from the start of docking of the patient bed 110 and the medical device 160 to the completion of docking. For example, the docking structure of the patient bed 110 and the medical device 160 may be screwed, and the preset depth may be the distance that the screw is screwed into the threaded hole. For another example, the docking structure of the patient bed 110 and the medical device 160 may adopt a helical connection, and the preset depth may be a number of revolutions of the screw screwed into the threaded hole (the number of revolutions and a diameter of the screw may be used to indirectly derive the travel distance of the patient bed 110). For another example, the docking structures of the patient bed 110 and the medical device 160 may be connected using the magnetic positioning pin assembly, and the preset depth may be the depth of the magnetic pin hole.

In some embodiments, the medical device 160 may be disposed with a docking verification device, which is configured to for determine whether or not the patient bed 110 is successfully docked with the medical device 160. The docking verification device may be a device for detecting whether a physical connection, an electrical connection, and a signaling connection have been made between the patient bed 110 and the medical device 160. In some embodiments, the docking verification device may be a distance detection device, which determines whether the docking has been successful or not by measuring a value of the preset depth. In some embodiments, the docking verification device may also be a trigger switch. The trigger switch may be cut off when the patient bed 110 is successfully docked with the medical device 160, and a docking success message may be sent to the processing device 140. When the processing device 140 obtains the docking completion information, an indication of the next operation may be given. For example, when the processing device 140 obtains the docking completion information that the docking has been successful, the medical device 160 may begin to perform a medical test on the patient (e.g., the MRI device may start performing an MRI scan on the patient). When the processing device 140 obtains the docking completion information of the docking failure, the processing device 140 may control the patient bed 110 to re-dock (e.g., the patient bed 110 may be controlled to retreat a preset distance to initiate the docking again).

In some embodiments, a retention structure may also be provided on the medical device 160. The retention structure may be configured to securely connect the patient bed 110 and the medical device 160, for preventing the patient bed 110 from shifting or wobbling, etc., during subsequent medical tests, and for ensuring an accuracy of a medical test result. In some embodiments, the retention structure of the medical device 160 and the patient bed 110 may be a locking device provided on the docking structure. For example, when the retention structure between the medical device 160 and the patient bed 110 is a slideway slider assembly, the locking structure may be a locking pin perpendicular to the slideway. When the slider on the patient bed 110 follows the slideway on the medical device 160 to the preset depth, the locking pin may protrude from the slideway and snap into a matching pin hole on the patient bed 110, thereby realizing the fixed connection between the patient bed 110 and the medical device 160.

In some embodiments, the retention structure may also be disposed on the patient bed 110. For example, the locking device may be disposed on the moving wheels of the patient bed 110, and when the processing device 140 obtains the docking completion information between the patient bed 110 and the medical device 160, the processing device 140 may control the locking device to lock the moving wheels to realize a position fixation of the patient bed 110.

In some embodiments, when the retention structure locks the patient bed 110 and the medical device 160 to maintain a fixed connection, the mobility device 131 may no longer be able to control the movement of the patient bed 110. In some embodiments, after the medical test at the medical device 160 for the patient on the patient bed 110 ends, the processing device 140 may send an end-of-docking command to the mobile device 131. Then the mobile device 131 may control the patient bed 110 for movement, and use the remote control mode and/or the follow mode to move the patient bed 110 to an initial ward.

In some embodiments, the controlling method for docking the mobile patient bed with the medical device may further include: when the patient bed 110 dockingly moved to the preset depth of the medical device 160, the retention structure may be initiated to secure the docking structure on the patient bed 110 with the docking structure on the medical device 160.

In some embodiments, the controlling method for docking a mobile patient bed with a medical device may further include: after the processing device 140 obtains the docking completion information, the docking completion information may be output through a display device for prompt. The display device may be disposed on the medical device 160, on the terminal 130, or on other devices, which is not limited herein.

In some embodiments, the display device may output the docking completion information for prompt through a voice output. In some embodiments, the display device may also output the docking completion information for prompt by means of a red and green light output, such as, for example, a green light being on to indicate that the docking is successful, and, for example, a red light being on to indicate that the docking is failed.

In some embodiments, the patient bed 110 may further be disposed with a detection module. The detection module may be configured for detecting information related to the patient bed 110, and the processing device 140 may analyze the information related to the patient bed 110 to process the information for any abnormality, and may give a prompt and/or a correction based on the detected abnormality.

In some embodiments, the detection module may detect the information related to the patient bed 110 in real time and send the information related to the patient bed 110 to the processing device 140. In some embodiments, the detection module may also detect the information related to the patient bed 110 according to a preset interval (e.g., 1 second, 2 seconds, or 3 seconds, etc.) and send the information related to the patient bed 110 to the processing device 140.

In some embodiments, the information related to the patient bed 110 may include a movement parameter, the traffic information, a state of the patient bed itself, etc., of the patient bed 110. The movement parameter may include a current position, a distance of movement, a direction of movement, a time of movement, a speed of movement, etc., or a combination thereof (see the preceding section for details). The traffic information may include obstacle information, road information, etc., or combinations thereof, in the direction of movement of the patient bed 110 (see previous section for details). The state of the patient bed itself may include the operating states of a driving motor of the driving device, an elevation motor used to raise the patient bed, a steering driving motor used to steer the patient bed, and/or other components, etc. of the patient bed 110.

In some embodiments, the prompt may be an alarm (e.g., a simple voice alarm that does not include a specific display of abnormality information). In some embodiments, the prompt may be an alarm alert (e.g., in a form of an image, in a form of a voice announcement, etc.) of specific abnormality information. In some embodiments, the prompt may be realized by a display device on the patient bed 110. In some embodiments, the prompt may also be realized through the display device on the mobile device 131. In some embodiments, the prompt may adopt the form of a voice, a flash, a vibration, or a combination of one or more forms.

The correction may include a correction advice and/or a correction implementation.

The correction advice may be a processing advice given by the processing device 140 based on the abnormity information obtained by the detection module for the patient bed 110 to resolve or avoid a damage from the abnormity information. In some embodiments, when the detection module detects an abnormality in a movement parameter of the patient bed 110, the detected abnormality information may be sent to the processing device 140, and the processing device 140 may control the patient bed 110 (or the terminal 130) to issue an alarm alert. In some embodiments, the processing device 140 may give the correction advice for the patient bed 110 based on the abnormality information detected by the detection module.

The correction implementation may be based on the abnormality information of the patient bed 110 obtained by the detection module, and the processing device 140 may calculate to obtain a correction plan and implement the correction plan to resolve or avoid the damage caused by the abnormality information.

In some embodiments, when the detection module detects that the movement of the patient bed 110 is too slow or the patient bed 110 stops, or a change in the movement distance is too small, etc., it indicates that the movement of the patient bed 110 is limited or trapped in a certain position. Specifically, when the movement of the patient bed 110 is too slow, the traffic information may indicate an uphill path, and the processing device 140 may send the alarm alert based on the movement parameter and the traffic information of the patient bed 110, and a correction advice to increase a driving force may be given. The operator may choose whether or not to correct based on the correction advice. In some embodiments, the processing device 140 may issue the alert prompt while making a correction directly based on the correction device obtained by calculation. For example, when an increase in drive force is required, the processing device 140 may directly control the driving device of the patient bed 110 to increase the driving force, without a need of manually selecting the correction plan, a purpose of increasing the movement speed of the patient bed 110 may be achieved.

In some embodiments, when the detection module detects an abnormality in the path information of the patient bed 110 (e.g., an obstacle exists in the direction of movement of the patient bed 110), the processing device 140 may issue the alarm alert based on the abnormality information (e.g., the height and/or the category of the obstacle) and recalculate to obtain a correction advice for one or more obstacle avoidance paths (e.g., the automatically planned paths or the preset paths). The operator may select a new path from the correction advice, and the processing device 140 may control the movement of the patient bed 110 in accordance with the new path to implement a path correction. In some embodiments, the processing device 140 may recalculate a reasonable automatically planned path for the patient bed 140 based on the abnormality information of the patient bed 110. The automatically planned path may avoid obstacles to ensure that the patient bed 110 moves quickly and smoothly toward the medical device 160. For another example, the processing device 140 may retrieve, based on the abnormality information of the patient bed 110, a suitable preset path from the storage device 150 and send the suitable preset path to the patient bed 110, and control the patient bed 110 to follow the new preset path to move towards the medical device 160.

In some embodiments, the detection module may detect the abnormality in the state of the patient bed 110 itself, and the processing device 140 may give an alert and/or correction based on the abnormality information. In some embodiments, the module may detect that a power storage of the driving device of the patient bed 110 is low, and the processing device 140 may give a prompt of a power shortage based on the power abnormality information and give a correction advice to go to the nearest charging station for a power change or a charging. A power storage minimum value may be preset within the processing device 140, and when the detected power storage is less than the power storage minimum value, it indicates that the power storage of the driving device is insufficient, that is, the detection module detects power abnormality information In some embodiments, the module may detect that the power storage of the driving device of the patient bed 110 is insufficient, and the processing device 140 may calculate, based on the power abnormality information, to obtain the automatically planned path to the charging station, and control the patient bed 110 to travel to the charging station to perform the correction plan of power exchange or charging.

In some embodiments, the module may detect that parts of the driving motor of the driving device of the patient bed 110 are damaged and is unable to drive the patient bed 110 to move normally, that is, the detection module detects the power abnormality information, and the processing device 140 may give, based on the power abnormality information, a prompt, and a correction advice for timely repairing the driving motor. In some embodiments, when the module detects that the parts of the driving motor of the driving device of the patient bed 110 are damaged, the processing device 140 may give the alert and stop the movement of the patient bed 110. In some embodiments, when the module detects that the parts of the moving wheel of the patient bed 110 are damaged, and the patient bed 110 is unable to steer flexibly, i.e., when the detection module detects moving wheel abnormality information, the processing device 140 may give, based on the moving wheel abnormality information, a prompt, and a correction advice to repair, or replace the moving wheel timely. In some embodiments, when the module detects that the parts of the moving wheel of the patient bed 110 are damaged, a prompt may be given, and the movement of the patient bed 110 may be stopped.

In some embodiments, after the patient on the patient bed 110 finishes the medical test at the medical device 160, the patient bed 110 may also automatically return to the initial position, and the processing device 140 may obtain, based on a patient bed code of the patient bed 110, the positioning information of the initial position of the patient bed 110 and control the return of the patient bed 110.

It should be noted that the foregoing descriptions of the process 200 is intended to be exemplary and illustrative only, and does not limit the scope of application of the present disclosure. For those skilled in the art, various corrections and changes may be made to the process 200 under the guidance of the present disclosure. However, these corrections and changes remain within the scope of the present disclosure. For example, the mobile device 131 may also be provided with a booster operation knob, and the operator may toggle the booster operation knob during the movement of the patient bed 110 to switch the movement mode of the patient bed 110, i.e., toggle the movement mode of the patient bed 110 from a current mode (i.e., the automatic mode) to a booster mode (i.e., a manual mode), at which time the operator is able to push the patient bed 110 to move the patient bed 110 through the knob on the patient bed 110.

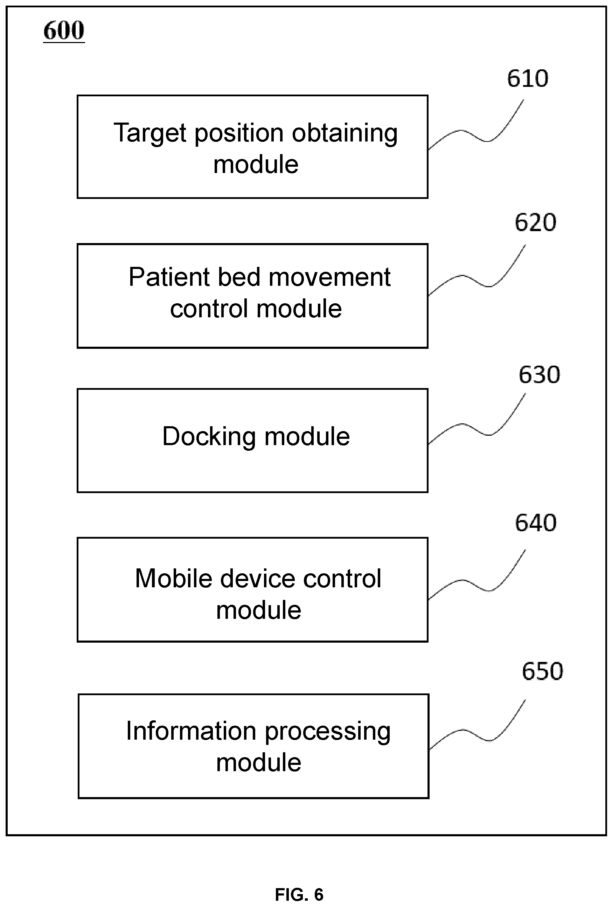

FIG. 6 is a schematic diagram illustrating a structure of a controlling system according to some embodiments of the present disclosure.

As shown in FIG. 6, a controlling system 600 for a mobile patient bed and a medical device may include the target position obtaining module 610, the patient bed movement control module 620, and the docking module 630. In some embodiments, the target position obtaining module 610, the patient bed movement control module 620, and the docking module 630 may be disposed on the patient bed 110. In some embodiments, the target position obtaining module 610 and the docking module 630 may also be disposed on the medical device 160, and the patient bed movement control module 620 may be disposed on the patient bed.

The target location obtaining module 610 may be configured to obtain positioning information of a target based on a docking instruction. In different embodiments, the positioning information of the target may include the positioning information of the patient bed 110, the positioning information of the mobile device 131, and/or the positioning information of the medical device 160, etc.

The patient bed movement control module 620 may be provided on the patient bed 110 and may be configured to control the movement of the patient bed 110 based on the positioning information of the target.

The docking module 630 may be configured to control a docking of the patient bed 110 with the medical device 160 when the patient bed 110 moves into a docking region of the medical device 160 guided by a docking structure.

In some embodiments, the controlling system 600 may further include a mobile device control module 640. The mobile device control module 640 may be provided on the mobile device 131. The mobile device control module 640 may control the patient bed 110 to follow the movement of the mobile device 131 by inputting operation instructions.

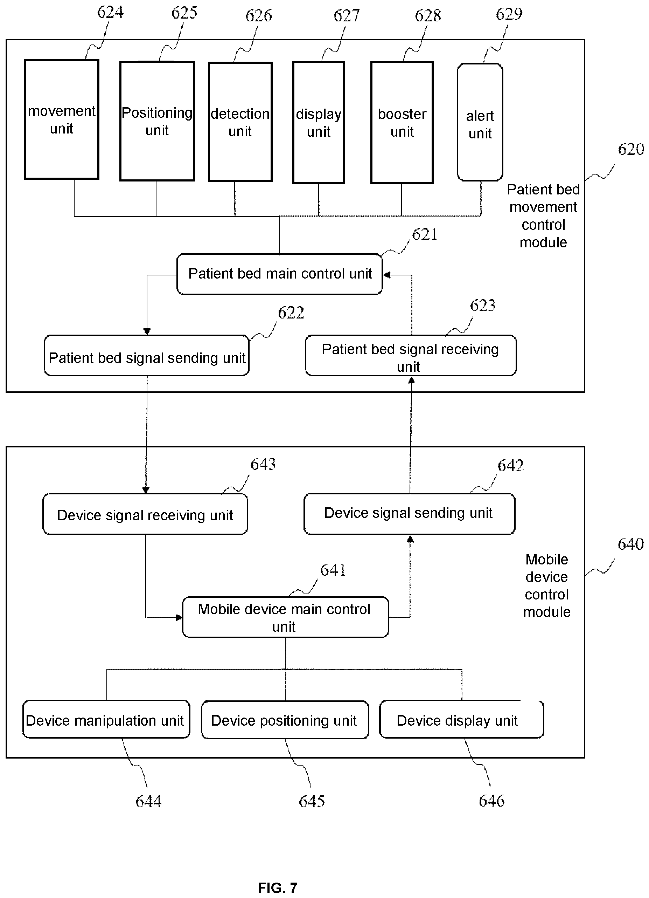

In some embodiments, as shown in FIG. 7, the patient bed movement control module 620 may include a patient bed main control unit 621, a patient bed signal sending unit 622, a patient bed signal receiving unit 623, a movement unit 624, a positioning unit 625, a detection unit 626, and/or a display unit 627. The patient bed main control unit 621 may be a main controller of the patient bed, may be in signal connections with various other sub-units, and may receive signals and process the signals and may send instructions to control the other sub-units. The patient bed signal sending unit 622 may be configured to send information related to the patient bed 110 to other modules (e.g., the target position obtaining module 610 or the docking module 630). The patient bed signal receiving unit 623 may be configured to receive the information sent to the patient bed 110 by other modules. The movement unit 624 may control the driving device of the patient bed 110 to implement a movement of the patient bed 110. The positioning module 625 may be configured to detect a real-time position of the patient bed 110. The detection module 626 may be configured for detecting the information related to the patient bed 110 (e.g., a movement parameter, a traffic information, a state of the patient bed itself). The display unit 627 may be configured to display the information related to the patient bed 110 (e.g., a current position, a running path, a preset path, a running speed, and/or traffic information).

In some embodiments, the patient bed movement control module 620 may further include a booster unit 628. The patient bed 110 may be converted to a human driven mode, and when the operator pushes the patient bed using the human driven mode (i.e., manually control the running direction of the patient bed 110), the booster unit 628 may control the driving device to provide a travel assist.

In some embodiments, the patient bed movement control module 620 may further include an alert unit 629. The alert unit 629 may issue an alert prompt based on the abnormality information of the patient bed 110.

In some embodiments, as shown in FIG. 7, the mobile device control module 640 may include a mobile device main control unit 641, a device signal sending unit 642, a device signal receiving unit 643, a device manipulation unit 644, a device positioning unit 645 and/or a device display unit 646. The mobile device main control unit 641 may be a main controller of the mobile device 131 and may be in a signal connection with each of the other sub-unit, so as to receive signals and perform signal process, and send instructions to control the other sub-units. The device signal sending unit 642 may be configured to send information related to the mobile device 131 to other modules (e.g., the target position obtaining module 610 or the patient bed movement module 620). The device signal receiving unit 643 may be configured to receive the information sent to the mobile device 131 by other modules. The device manipulation unit 644 may obtain a manipulation instruction through manipulation buttons (e.g., various buttons such as front, back, left, right, start, stop, accelerate, decelerate, etc.). The device positioning unit 645 may be configured to detect a real-time position of the mobile device 131. The device display unit 646 may be configured to display relevant information (e.g., a current position, a running path, a preset path, a running speed, and/or traffic information, etc.) of the mobile device 131.

In some embodiments, as shown in FIG. 7 of the accompanying drawings, the mobile device 131 may control the movement of the patient bed 110. For example, the mobile device 131 may be carried by the operator (e.g., the healthcare worker) to control the movement of the patient bed 110 to follow the movement of the operator. For the specific movement of the following movement, please refer to the embodiment above. As shown in FIG. 7, the mobile device 131 may obtain a manipulation designation through the device manipulation unit 644, and obtain the positioning information of the mobile device 131 through the device positioning unit 645. The mobile device main control unit 641 may process the manipulation instruction and the positioning information of the mobile device 131, and send the movement signal to the patient bed information receiving unit 623 through the device signal sending unit 642. The patient bed 110 may obtain the movement signal trough the patient bed information receiving unit 623. The patient bed main control unit 621 may control, by the movement unit, the patient bed 110 to follow the mobile device 131 to move based on the movement signal and the positioning information of the patient bed 110, etc.

It may be noted that the above description of the controlling system for docking the mobile patient bed with the medical device, and the device/module thereof are only for descriptive convenience, and do not limit the present disclosure to the scope of the cited embodiments. It is to be understood that for those skilled in the art, with an understanding of the principle of the system, it may be possible to arbitrarily combine individual devices/modules or form sub-systems to connect with other devices/modules. For example, in some embodiments, the target position obtaining module 610, the docking module 630, and the main control module 640 disclosed in FIG. 6 may be different modules in a single device (e.g., the processing device 140), and may also be a single module that implements the functions of two or more of the modules described above. For example, in some embodiments, the patient bed signal receiving unit 623 and the patient bed signal sending unit 622 in FIG. 7 may be two units, or a single unit may have both signal receiving and signal transmission functions. For example, the individual modules may each have a respective storage module. For another example, each module may share a storage module. Modifications like these are within the scope of protection of the present disclosure.

In some embodiments, the controlling device for docking the mobile patient bed and the medical device may include at least one processor and at least one memory. The at least one memory may be configured to store computer instructions. The at least one processor may be configured to execute at least a portion of these computer instructions to implement the controlling method for docking the mobile patient bed and the medical device of any of the above embodiments.

Some embodiments provide a non-transitory computer readable storage medium storing computer instructions, wherein when executing the computer instructions in the non-transitory computer readable storage medium, a computer implements a controlling method for docking a mobile patient bed with a medical device.

Beneficial effects that may be brought about by the embodiments of the present disclosure may include, but not limited to: (1) enabling the patient bed to automatically move to the scanning room to realize the docking with the medical device, freeing the operator's hands, which relieves the operator's toil and improves an operation efficiency; (2) by switching the movement control mode of the patient bed (e.g., an automatic planning path mode, a preset path mode, a following mode, etc.), the operator may control the movement of the medical device flexibly, and improve an operator experience; (3) the patient bed may automatically realize the docking with the medical device through the docking structure without manual alignment, thereby reducing the corresponding operation error, and saving a docking time.

It should be noted that the beneficial effects generated by different embodiments may be different, and the beneficial effects generated in different embodiments may be any one or a combination of the foregoing, or any other beneficial effect that may be obtained.

The basic concepts have been described above, and it is apparent to those skilled in the art that the foregoing detailed disclosure is intended as an example only and does not constitute a limitation of the present disclosure. While not expressly stated herein, various modifications, improvements, and amendments may be made to the present disclosure by those skilled in the art. These types of modifications, improvements, and amendments are suggested in the present disclosure, so these types of modifications, improvements, and amendments remain within the spirit and scope of the exemplary embodiments of the present disclosure.

Also, the present disclosure uses specific words to describe embodiments thereof. such as "an embodiment," "one embodiment," and/or "some embodiment" means a feature, structure, or characteristic associated with at least one embodiment of the present disclosure. Accordingly, it should be emphasized and noted that the "an embodiment," "one embodiment," or "an alternative embodiment" referred to two or more times in different positions in the present disclosure do not necessarily refer to the same embodiment. In addition, certain features, structures, or characteristics in one or more embodiments of the present disclosure may be suitably combined.

Additionally, unless expressly stated in the claims, the order of the processing elements, the use of numerical letters, or the use of other names as described in the present disclosure are not intended to limit the order of the processes and methods of the present disclosure. While some embodiments of the present disclosure that are currently considered useful are discussed in the foregoing disclosure by way of various examples, it should be appreciated that such details serve only illustrative purposes, and that additional claims are not limited to the disclosed embodiments. Rather, the claims are intended to cover all amendments and equivalent combinations that are consistent with the substance and scope of the embodiments of the present disclosure.

Similarly, it should be noted that in order to simplify the presentation of the disclosure of the present disclosure, and thereby aiding in the understanding of one or more embodiments of the present disclosure, the foregoing descriptions of embodiments of the present disclosure sometimes group multiple features together in a single embodiment, accompanying drawing, or descriptions thereof. However, this mode of disclosure does not imply that the objects of the present disclosure require more features than those mentioned in the claims.

Some embodiments use numbers to describe the number of components and attributes, and it should be understood that such numbers used in the description of the embodiments are modified in some examples by the modifiers "about," "approximately," or "substantially". Unless otherwise noted, the terms "about," "approximate," or "substantially" indicates that a ±20% variation in the stated number is allowed. Correspondingly, in some embodiments, the numerical parameters used in the present disclosure and the claims are approximations, which change depending on the desired characteristics of individual embodiments. In some embodiments, the numerical parameters should consider the specified number of valid digits and employ a general place-keeping. While the numerical domains and parameters used to confirm the breadth of their ranges in some embodiments of the present disclosure are approximations, in specific embodiments such values are set to be as precise as possible within the feasible range.

Finally, it should be understood that the embodiments described herein are only used to illustrate the principles of the embodiments of the present disclosure. Other deformations may also fall within the scope of the present disclosure. As such, alternative configurations of embodiments of the present disclosure may be viewed as consistent with the teachings of the present disclosure as an example, not as a limitation. Correspondingly, the embodiments of the present disclosure are not limited to the embodiments expressly presented and described herein.

## Claims

1. A controlling method for docking a mobile patient bed with a medical device, comprising:
obtaining, based on a docking instruction, positioning information of a target;
controlling, based on the positioning information of the target, a movement of the mobile patient bed;
in response to determining that the mobile patient bed moves into a docking region of the medical device, controlling the mobile patient bed to dock with the medical device under a guidance of at least two docking structures.

2. The controlling method of claim 1, wherein the positioning information of the target includes positioning information of the medical device, and
the controlling, based on the positioning information of the target, the movement of the mobile patient bed comprises:
obtaining an automatically planned path based on the positioning information of the medical device, and moving the mobile patient bed along the automatically planned path; or
moving the mobile patient bed based on the positioning information of the medical device and a preset path.

3. The controlling method of claim 1, wherein the positioning information of the target includes positioning information of a mobile device, and
the controlling, based on the positioning information of the target, the movement of the mobile patient bed comprises:
controlling, based on the positioning information of the mobile device, the mobile patient bed to follow the mobile device.

4. The controlling method of claim 3, wherein the controlling, based on the positioning information of the mobile device, the mobile patient bed to follow the mobile device comprises:
determining, based on the positioning information of the mobile patient bed and the positioning information of the mobile device, a movement parameter of the mobile patient bed.

5. The controlling method of claim 3, wherein
in response to determining that the mobile patient bed is outside a guiding region, control the mobile patient bed to follow the mobile device based on the positioning information of the mobile device; and
in response to determining that the mobile patient bed is moved into the guiding region, controlling the mobile patient bed to move based on the positioning information of the medical device.

6. The controlling method of claim 3, wherein
in response to determining that the mobile patient bed is outside the guiding region, control the mobile patient bed to follow the mobile device based on the positioning information of the mobile device; and
in response to determining that the mobile patient bed moves into the guiding region, obtaining the positioning information of the mobile patient bed through a wireless positioning module, and controlling the mobile patient bed to move based on the positioning information of the mobile patient bed.

7. The controlling method of claim 2, wherein the obtaining the automatically planned path based on the positioning information of the medical device comprises:
capturing, by a camera, an optical image including a surrounding of the mobile patient bed; and
obtaining, by a path planning module, the automatically planned path based on the optical image.

8. The controlling method of claim 2, wherein the obtaining the automatically planned path based on the positioning information of the medical device comprises:
sensing, by a plurality of sensors, traffic information around the mobile patient bed; and
obtaining, by a path planning module, the automatically planned path based on the traffic information.

9. The controlling method of claim 1, further comprising:
displaying, by a display device, a current movement position of the mobile patient bed.

10. The controlling method of claim 1, further comprising:
in response to determining that the mobile patient bed moves into the docking region of the medical device and a first docking structure on the mobile patient bed starts to dock with a second docking structure on the medical device, controlling the second docking structure on the medical device and the first docking structure on the mobile patient bed to actively drag the mobile patient bed towards the medical device.

11. The controlling method of claim 1, further comprising:
in response to determining that the mobile patient bed is moved to a track within the docking region of the medical device, controlling, by the track, the mobile patient bed to dock with the medical device.

12. The controlling method of claim 10 or claim 11, further comprising:
in response to determining that the mobile patient bed moves to a preset depth of the medical device, obtaining docking completion information between the mobile patient bed and the medical device.

13. The controlling method of claim 12, further comprising:
in response to determining that the mobile patient bed moves to the preset depth of the medical device, fixing the first docking structure on the mobile patient bed and the second docking structure on the medical device by initiating a retaining structure.

14. The controlling method of claim 1, further comprising:
detecting, by a detection module, information related to the mobile patient bed, and
alerting and/or correcting abnormal information of the detected information related to the mobile patient bed.

15. A controlling system for docking a mobile patient bed with a medical device, comprising:
a target positioning information obtaining module configured to obtain, based on a docking instruction, positioning information of a target;
a bed movement control module configured to control, based on the positioning information of the target, a movement of the mobile patient bed; and
a docking module configured to control the mobile patient bed to dock with the medical device under a guidance of at least two docking structures in response to determining that the mobile patient bed moves into a docking region of the medical device.

16. A controlling device for docking a mobile patient bed with a medical device, comprising at least one processor and at least one storage device;
the at least one storage device being configured to store computer instructions;
the at least one processor being configured to perform at least a portion of the computer instructions to implement a controlling method for docking a mobile patient bed with a medical device of any one of claims 1 to 14.

17. A non-transitory computer readable storage medium storing computer instructions, wherein when executing the computer instructions in the non-transitory computer readable storage medium, a computer implements a controlling method for docking a mobile patient bed with a medical device of any one of claims 1 to 14.
